# EUROPEAN PATENT SPECIFICATION

(11) **EP 3 434 292 B1**
(45) Date of publication and mention of the grant of the patent: **23.08.2023**
(21) Application number: 18185629.5
(22) Date of filing: 25.07.2018
(51) Int. Cl.: A61L 27/24, A61L 27/48, A61L 27/50, A61L 27/58

(54) **COMPOSITE BLOOD VESSEL SUBSTITUTE AND THE METHOD FOR PRODUCING IT**
ZUSAMMENGESETZTER BLUTGEFÄSSERSATZ UND VERFAHREN ZUR HERSTELLUNG DAVON
SUBSTITUT DE VAISSEAU SANGUIN COMPOSITE ET SON PROCÉDÉ DE PRODUCTION

(30) Priority: 26.07.2017 CZ 20170427
(43) Date of publication of application: 30.01.2019
(73) Proprietor: Ceske vysoke uceni technicke v Praze, 160 00 Praha 6 - Dejvice (CZ); Vseobecná fakultní nemocnice v Praze, 12800 Praha 2, Nové Mesto (CZ); Univerzita Karlova, 128 00 Praha 2, Nové Mesto (CZ); Vyzkumny Ustav Potravinarsky Praha, V.V.I., 10200 Praha 10, Hostivar (CZ)
(72) Inventor: CHLUP, Hynek, 79807 Dobrochov (CZ); GRUS, Tomás, 25210 Mnísek pod Brdy (CZ); MLCEK, Mikulás, 10000 Praha 10, Strasnice (CZ); BERAN, Milos, 26301 Dobrís (CZ); SPACEK, Miroslav, 12800 Praha 2, Nové Mesto (CZ)
(74) Representative: Lunzarová, Lucie

(56) References cited:
- EP-A1- 0 493 788
- EP-A1- 1 618 856
- EP-A2- 0 518 389
- JP-A- 2017 047 031
- US-A1- 2014 242 140

## Description

### Field of the Invention

The present invention relates to a composite tube with a biological and non-biological component intended as an artificial vascular replacement, with an inner diameter less than 6 mm, and for flow rates under 100 ml/min, withstanding inner pulse pressures of up to 420 mmHg (about 56 kPa), and a method for producing it.

### Background of the Invention

Synthetic blood vessel prostheses using polyethylene terephthalate (PET, Dacron) and expanded polytetrafluoroethylene (ePTFE) are known from previous practice. VUP Brno, where prostheses based on collagen-coated polyester silk have been developed since 1950s, was and remains a great pioneer in the production of vascular substitutes in the Czech Republic. Collagen is a water-insoluble scleroprotein, which is the major component of skin, cartilages, bones, cornea, blood vessel walls, tendons and teeth - it makes up 25% of proteins in the bodies of mammals.

These polymers work well as substitutes for large-diameter blood vessels; however, their long-term patency in the replacement of small-diameter vessels (< 6 mm) is unsatisfactory. Patient's own artery or vein remains the substitute of choice in coronary, crural or microvascular surgery in general. Vascular prostheses fail due to thrombosis and insufficient healing process that consists in highly incomplete endothelial cell coverage and intimal hyperplasia caused by compliance mismatch and haemodynamic imbalance.

Known collagen prostheses contain collagen, a protein, which is a major structural component of connective tissue and internal organ extracellular mass and makes up 25-30% of all proteins in mammalian systems. There are at least 27 different types of collagen presently known, of which types I, II, III, IV and V are the most important. Type I collagen is present in the skin, bones, tendons and teeth, type II collagen is present in cartilages. Type III is embryonic development collagen, which is subsequently substituted by type I. Type IV is found in epithelial basal lamina, and type V collagen is typical of the blood vessel wall.

Type I is the most common collagen, makes up 90% of all collagen in the body, has a triple helix structure made up of three polypeptide strands rich in amino acids glycine, proline, hydroxyproline and hydroxylysine. The amino acid composition of collagen is similar in different animal species, and usually varies only slightly.

Warp-knit fabric venous prostheses are coated with a continuous film of chemically modified collagen.

In general terms, drawbacks of previous prostheses are as follows:
1) Limited use for low flow areas (crural arteries, coronary arteries);
2) Possibility of body's immune response, insufficient healing;
3) Susceptibility to venous prosthesis infection, with the need to remove the material from the body;
4) The issue of anastomotic hyperplasia;
5) The issue of thrombosis in venous prostheses.

Basic requirements for vascular substitutes include bio-compatibility, suitable mechanical properties - sufficient strength and viscoelastic properties similar to those of native vessels, and the capability to adapt to the changing blood flow conditions. Another essential requirement is a low thrombogenicity of the vascular substitute's inner surface (Sarkar, et al, 2007). Highest requirements are put on low-flow vascular substitutes, which are very prone to clogging. No truly reliable synthetic vascular substitutes for these low flows are currently available on the market. Chan-Park, et al. (2009) describe tissue engineering procedures leading to desired vascular substitute properties. In addition, requirements for biodegradable materials are discussed here. Low-flow vascular substitutes are urgently required especially for patients who need coronary and popliteal vessel replacement. Current therapies include the use of autologous vessels and synthetic substitutes (Nerem, Seliktar, 2001). Sarkar, et al., 2007 deal with required mechanical properties of infrainguinal bypass grafts. An ideal biomaterial for this area of tissue engineering should not only approach native vessels with its mechanical properties but it should also support cell growth, support extracellular matrix production, and inhibit thrombogenicity (Ravi and Chaikof, 2010).

Kaibara, et al. (1995) described the *in vitro* method of biomaterial thrombogenicity evaluation.

Similar known solutions have been described in the following papers.

M.B. Chan-Park, J.Y. Shen, Y. Cao, Y. Xiong, Y. Liu, S. Rayatpisheh, G.C. Kang, H.P. Greisler. Biomimetic control of vascular smooth muscle cell morphology and phenotype for functional tissue-engineered small-diameter blood vessels. J Biomed Mater Res A. 91 (2): 629-34 (2009).

H. Haruguchi, S. Teraoka. Intimal hyperplasia and hemodynamic factors in arterial bypass and arteriovenous grafts: a review. J Artif Organs. 6(4):227-35 (2003).

T.L. Johnson, G.A. Barabino, R.M. Nerem. Engineering more physiologic in vitro models for the study of vascular biology. Progress in Pediatric Cardiology 21: 201 - 210 (2006).

M. Kaibara, Y. Kawamoto, S. Yanagida, S. Kawakami. In vitro evaluation of antithrombogenicity of hybrid-type vascular vessel models based on analysis of the mechanism of blood coagulation. Biomaterials 16: 1229-1234 (1995).

Gregor Knöner, Barbara E. Rolfe, J.H. Campbell, S.J. Parkin, N.R. Heckenberg, H. Rubinsztein-Dunlop. Mechanics of Cellular Adhesion to Artificial Artery Templates. Biophysical Journal Volume 91: 3085-3096 (2006).

R.M. Nerem. Tissue engineering a blood vessel substitute: the role of biomechanics. Yonsei Med J. 41 (6): 735-9 (2000).

R.M. Nerem. Role of mechanics in vascular tissue engineering. Biorhelogy 40 (1-3): 281-7 (2003).

R.M. Nerem. Critical issues in vascular tissue engineering. International Congress Series 1262: 122-125 (2004).

R.M. Nerem, D. Seliktar. Vascular tissue engineering. Annu Rev Biomed Eng. 3:225-43 (2001).

S. Ravi, E.L. Chaikof. Biomaterials for vascular tissue engineering. Regen Med. 5 (1): 107 (2010).

S. Sarkar, H.J. Salacinskij, G. Hamilton, A.M. Seifalian. The mechanical properties of infrainguinal vascular bypass grafts: their role in influencing patency. Eur J Vasc Endovasc Surg. 31(6):627-36 (2006).

S. Sarkar, T. Schmitz-Rixen, G. Hamilton, A.M. Seifalian. Achieving the ideal properties for vascular bypass grafts using a tissue engineered approach: a review. Med Biol Eng Comput. 45 (4): 327-36 (2007).

Z. Yang, J. Tao, J.-M. Wang, Ch. Tu, M.-G. Xu, Y. Wang, S.-R. Pan. Shear stress contributes to t-PA mRNA expression in human endothelial progenitor cells and nonthrombogenic potential of small diameter artificial vessels. Biochemical and Biophysical Research Communications 342: 577-584 (2006).

In addition, commercial non-biodegradable and biodegradable materials are known.

### Commercial synthetic non-biodegradable materials

Harrison (1958) published a survey and a comparative study of synthetic materials used to produce large-diameter vascular prostheses - nylon, Dacron, Orlon, Ivalon (PVAc) and teflon. Commercial synthetic non-biodegradable materials also include polytetrafluoroethylene (PTFE), various types of polyester or GORETEX. These are rigid materials that are unsuitable for low-flow vascular constructs (Tiwari, et al., 2002). Schutte and Nerem (2013) published a detailed survey of materials used to produce vascular prostheses, including overview tables of materials used with references to literature. Evansen (2011) deals with vascular substitute market analysis in his bachelor's thesis.

### Commercial biodegradable materials

Collagen is a biopolymer most commonly used for vascular constructs. Khan, et al. (2011) in their overview describe the use of collagen for vascular substitute production in other areas of reconstructive medicine.

These materials are described in the following publications.

B.E. Evansen. Market and Business Analysis of Tissue Engineered Blood Vessels. A Major Qualifying Project Report Submitted to the Faculty of the Worcester Polytechnic Institute in partial fulfillment of the requirements for the Degree of Bachelor of Science in Management Engineering with a Biomedical Engineering Concentration. April 2011.

J.H. Harrison. Synthetic materials as vascular prostheses: II. A comparative study of nylon, dacron, orlon, ivalon sponge and teflon in large blood vessels with tensile strength studies. The American Journal of Surgery 95 (1): 16-24 (1958).

Y.P. Jiao, F.Z. Cui. Surface modification of polyester biomaterials for tissue engineering. Biomed Meter. Biomed Mater. 2(4):R24-37 (2007).

R. Khan, M.H. Khan, A. Bey. Use of collagen as an implantable material in the reconstructive procedures - an overview. Biology and Medicine, 3 (4): 25-32 (2011).

S. Roll, J. Müller-Nordhorn, T. Keil, H. Scholz, D. Eidt, W. Greiner, S.N. Willich. Dacron vs. PTFE as bypass materials in peripheral vascular surgery--systematic review and meta-analysis. BMC Surg. 8: 22 (2008).

S.C. Schutte, R.M. Nerem. CHAPTER II.6.9 BLOOD VESSEL TISSUE ENGINEERING.

From: B.D. Rarner, A.S. Hofman, F.J. Schoen, J.E. Lemons (editors). Biomaterials Science: An Introduction to Materials in Medicine. Academic Press; 3 edition, January 2013.

A. Tiwari, H. Salacinski, A.M. Seifalian, G. Hamilton. New prostheses for use in bypass grafts with special emphasis on polyurethanes. Cardiovasc Surg 10(3):191-7 (2002).

X. Wang, P. Lin, Q. Yao, C. Chen. Development of small-diameter vascular grafts. World J Surg 31(4):682-9 (2007).

Below is an overview of methodologies and materials subject to research and development.

### Overview of methodologies used for vascular constructs

A wide range of procedures and materials are currently tested for the preparation of biologically functional vascular substitutes. These methods include vascular substitute construction using natural gels and synthetic scaffolds populated by living vascular tissue cells and acellular technique (refer to the overview paper by Shaikh, et al., 2008).

Son and Kim (2009) describe the preparation of polymeric scaffolds with microstructured surface using 3D printing. The use of 3D printing procedures ("rapid prototyping") for tissue engineering is also the subject of other papers (Peltola, et al., 2008).

Sarkar, et al. (2009) used a new automatic extrusion method ("extrusion-phase-inversion method") to fabricate low-flow vascular substitutes from polymer nanocomposite containing polycarbonate urethane and polyhedral oligomeric silsesquioxane. The specimens thus produced had good antithrombogenic properties and bistability.

Lovett, et al. (2008; 2010) described the process of tissue substitute fabrication from silk fibroin by gel fibre spraying on a rotating shaft ("gel spinning"). When tested *in vitro* and *in vivo,* the fabricated vascular substitutes outperformed commercial PTFE vascular prostheses in some aspects.

Singha, et al. (2012) published an overview of methods and materials to fabricate vascular prostheses in the commercial and research spheres.

Chaouat, et al. (2008) described the preparation of PVA low-flow vascular substitutes by simple wrapping dissolved materials around a Teflon rod.

The overview paper by Kizildag and Yalcin (2012) deals with the possibilities of using electrospun nanofibre structures for vascular prosthesis fabrication.

Vaz, et al. (2005) described the construction of vascular prostheses with morphological architecture mimicking native blood vessels by use of wrapping variously oriented electrospun PLA and PCL nanofibres around a collector in the form of a rotating shaft. The construction of vascular prostheses using combined electrospinning and L-lactide and caprolactone copolymer melt spinning technology is described in the article by Chung, et al., 2010. Widmer, et al. (1998) used extrusion technology to fabricate tubular scaffolds from the mix of PLGA and PLA. Skardal, et al. (2010) used hyaluronic acid hydrogels crosslinked with tetrahedral PEG-tetraacrylates for 3D printing vessel-like constructs. Electrohydrodynamic atomization procedures have also been used for vessel substitute fabrication (Stankusa, et al., 2007). Lee, et al. (2009) used a polymer prepared by the poly-condensation of malic acid and 1,12-dodecandiol and a method for producing foams using supercritical carbon dioxide to prepare biomaterials for vascular prostheses.

### Biopolymer implants and implants prepared using tissue engineering methods

Kannan, et al. (2005) published a list of new, successfully tested materials for vascular constructs of biological and synthetic origin, particularly for use in by-passes.

Kakou, et al. (2007) published an overview of materials tested for fabricating scaffolds for vascular tissue engineering. Some of them, such as collagen, are also used commercially. Biomaterials of biological origin under intensive research include the protein fibrin found in native blood vessels like collagen. Fibrin has excellent mechanical properties and biocompatibility but its high price is a disadvantage. Jiang, et al., 2008 deal with the possibilities of using chitosan in tissue engineering and regenerative medicine in their overview paper.

Van Alst, et al., 2009 describes the application of polylactic acid to create fully bioabsorbable stents.

Nemeno-Guanzon, et al., 2012 comprehensively describe new trends in tissue engineering.

Encouraging results have been achieved by Kong, et al., (2012) who tested vascular prostheses for low blood flows prepared from biodegradable chitosan.

Vrana, et al. (2010) tested the effect of shear stress on endothelised composite hydrogels prepared from PVA and gelatin.

Elastin is a key structural matrix protein that provides it with elasticity. In addition, it has many regulatory functions and influences cell phenotypes and activities. The function of elastin has been mostly underestimated in tissue engineering (Patel, et al., 2006). Koens, et al. (2010) described the process to produce 3-layered vascular grafts containing collagen and elastin.

Ravi and Chaikof (2010) published an overview of biomaterials used for blood vessel tissue engineering. An ideal biomaterial for this area of tissue engineering should not only approach native vessels with its mechanical properties but also support cell growth, support extracellular matrix production, and inhibit thrombogenicity. Another overview of materials for blood vessel tissue engineering was published by Chlupá 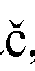 , et al. (2009). Bacterial cellulose was used by Klemm, et al., 2001 to prepare vascular substitutes for microsurgery. Fink (2009) in his doctoral thesis deals with the possibilities of using bacterial cellulose for vascular constructs, especially in terms of this biopolymer interactions with blood and endothelial cells. Blood vessel tissue engineering for low flows and biomaterials used are addressed by Heyligers et al. (2005) in their overview article. Silk fibroin is a very promising material for low-diameter vascular constructs (Lovett, et al., 2007). Xiang, et al. (2011) used composite nanofibres containing recombinant spider silk protein, PCL and gelatin to prepare tubular scaffolds.

### Tested synthetic materials for vascular constructs

Schmedlen, et al., 2003 published an overview of synthetic biomaterials used for vascular tissue engineering. Tiwari, et al. (2002) published an overview paper focused on the possibility of using non-biodegradable polyurethane materials for low-flow vascular constructs. This type of materials seems to be very promising.

Oligomeric silsesquioxane nanocomposites are a relatively new biomaterial tested as vascular constructs (Solouk, et al., 2011; Cozza, et al., 2012).

Domurado, et al. (1978) showed that the properties of knitted dacron vascular substitutes, including prosthesis population with cells and collagen encapsulation development, improved when impregnated with albumin. Schneider, et al. (1993) describe the procedure of goretex vascular substitute treatment using fibrin and/or extracellular matrix, with subsequent population with bovine aorta endothelial cells. Elshazly (2004) in his doctoral thesis deals with using potentially biodegradable PVA hydrogels for vascular constructs.

Izhar, et al. (2001) tested low-flow vascular prostheses prepared from PU fibres (Lycra) with biodegradable PELA (poly(ethylene glycol)/poly(lactic acid)) coat with very good results. Niekraszewicz, et al. (2009) improved the properties of vascular prostheses prepared from polyester knitted fabric by their impregnation with polymer *poly*[*D,L-(lactide-co-glycolide*)].

Fiorica, et al. (2012) prepared scaffolds from electrospun composite copolymer PE-PA fibres and immobilized heparin on their surface. Heparin binds several growth factors, including vascular endothelial growth factor (VEGF) and basic fibroblast growth factor (bFGF), thus increasing biomaterial population with relevant cells and improving their proliferation.

Millon, et al. (2012) functionalised physically crosslinked PVA with fibronectin in order to improve cell adhesion with proliferation of porcine arterial and vascular endothelial cells. The efficacy of functionalisation was confirmed by experimental testing. Choi and Noh (2005) functionalised ePTFE scaffolds with a biodegradable PLGA layer using ammonium carbonate as a porogen. To improve tissue regeneration, the PLGA surface was additionally coated with a gelatin layer.

Huanga, et al. (2011) used a spatially arranged eclectrospun mesh of collagen, chitosan and thermoplastic polyurethane nanofibres with subsequent glutaraldehyde crosslinking in order to mimic native extracellular matrix structure. Tests have shown a good biocompatibility of scaffolds. Spatial arrangement of nanofibres affects cell morphology.

Greislertt, et al. (1996) impregnated the ePTFE graft with fibrin adhesive containing FGF-1 growth factor and heparin. The authors have thus succeeded in increasing the proliferation of endothelial cells and smooth muscle cells following vascular substitute implantation in dogs.

Disadvantages of the solutions described include the price in the first place and non-availability of major part of the present vascular substitutes in clinical practice. For biomaterials providing higher proliferation of endothelial cells and smooth muscle cells, long-term testing of patency in clinical practice is missing, with respect to potential hyperplasia and partial obliteration of the discharge lumen, especially in prostheses designed for low-flow areas.

Vascular prostheses with diameters of at least 6 mm are commercially available. Prostheses with lower diameters that would be tested in this way and intended for low flows are unavailable yet.

EP 0 518 389 describes vascular grafts having an internal diameter of no more than 4 mm. Said graft comprises a non-resorbable synthetic substrate, which is embedded within one or more under-layers comprising collagen type I/III and a top layer comprising collagen type IV/V.

### Summary of the Invention

The above drawbacks have been largely remedied by the composite vascular substitute with inner diameters below 6 mm and flow rates under 100 ml/min of this technical solution. Hereby the present invention is defined by the independent claims, while the dependent claims concern the preferred embodiments. It essentially provides that the vascular substitute contains an inner resorbable layer of collagen compound overlaid with a non-resorbable knitted fabric layer covered by an external layer of collagen compound again. Or, the substitute is formed by one layer consisting of a resorbable collagen compound, in which a layer of knitted fabric is integrated during fabrication.

The knitted fabric is coated with the collagen compound. According to the claimed invention, the collagen compound to fabricate the composite tube (prosthesis) is obtained by separating fish collagen from the skin of the freshwater fish Czech carp. Therefore, we refer to the inner and outer layer or side of the vascular substitute. The outer layer/side is treated differently than the inner layer/side. This should ensure faster absorption of the outer collagen layer/side. During the process of prosthesis integration in the organism, the penetration of fibroblasts to the wall centre improves thus ensuring early healing of the graft. This appears as a major advantage of this prosthesis. In a preferred embodiment, the non-resorbable middle layer is made of polyester knitted fabric. One fibre of the non-resorbable layer consists of 2 to 5 monofilaments combined in one bundle before knitting. This spinning process provides the graft with suitable mechanical response to the pulsed blood flow. The graft wall is not rigid and is able to partially transfer the pulse wave while maintaining the integrity of the inner collagen layer/side. The knitted fabric is non-folded.

In addition, the present invention relates to the method for producing the vascular substitute. It essentially provides that the collagen compound with a collagen concentration of 4 to 12% by weight is kept at 15 to 30 °C for 18 to 30 hours, and then it is extruded to make the inner resorbable layer in the form of a tube that is dried at the room temperature until pliable. Then the inner resorbable layer is overlaid with the non-resorbable knitted fabric layer, which is covered with the outer resorbable layer of collagen compound again, with a collagen concentration of 3 to 12% by weight. The vascular substitutes are dried at 15 to 30 °C for 15 to 30 hours until pliable. Single-layer collagen composite substitutes are also preferably made by extruding the collagen compound with a collagen concentration of 4 to 12% by weight whereby the inner and the outer collagen sides of the prosthesis are extruded at the same time while continuously applying the collagen compound to the knitted fabric. The collagen compound is kept at same temperatures as in the previous case. Following one-time extrusion, the prosthesis is dried at 15 to 30 °C for 15 to 30 hours until pliable. The vascular substitutes are hardened with 1.5 to 3.2% resin for 3 to 10 minutes and then they are dried and softened up in a 15 to 30% glycerine bath for 15 to 25 minutes.

The collagen compound is preferably enriched with evenly dispersed pharmacologically effective substances including antibiotics, antiplatelet drugs or anticoagulants. For the laminated prosthesis, a 1 to 3% by weight of collagen compound solution can be applied between the inner resorbable layer and the non-resorbable layer.

Furthermore, the vascular prostheses are cut to length. The vascular substitute specimens are individually wrapped in double sterilization packaging and sterilized by standard radiation dose for medical products. For gamma radiation, for example, this dose ranges between 25 and 30 kGy.

As part of researching an ideal low-diameter vascular substitute with a inner diameter less than 6 mm, some surgical procedures were changed, new biomaterials developed, cell and tissue culture technology introduced and, last but not least, new types of vascular substitutes and materials were developed for their final treatment.

The new, unique procedure of collagen compound extrusion for the vascular prosthesis of this technical solution improves its properties in the area of low-flow artificial vascular substitutes with inner diameters under 6 mm, as proved on an animal model and described below. During extrusion, the collagen compound flows through a specially developed head, where the speed and direction of rotation around its axis, i.e. longitudinal axis of the extruded tube, can be controlled. During the extrusion process, the collagen fibre bundles are arranged (directed) in the flow direction in the originally homogenized collagen compound. This makes the isotropic material anisotropic. The special rotating head enables creating different pitch angles and thereby the inner helical structure of oriented collagen fibre bundles in the fabricated substitute wall. The principle consists in the control of head direction and rotations with respect to the extrusion speed. This generates the adequate structure/pitch of collagen fibre bundles. In addition, the right-hand and left-hand pitch and interlacing of fibres can be changed. This structure is similar to the inner structure of native human blood vessels, in which collagen fibres are arranged in this way. In native blood vessels, the orientation of the dominant direction of collagen fibres as well as the pitch of a notional helix may change in each layer: the intima, media and the adventitia. It is the composite, multilayer, sandwich structure of the alternative vascular substitute wall that can provide construction and functionality similar to native blood vessels. This procedure has been subsequently histologically verified by comparing it with the structure of collagen in human blood vessels. This procedure creates unique properties of the vascular substitute, particularly with respect to the pulse wave transfer, as further demonstrated on an animal experiment using ultrasound monitoring, see below.

The proposed vascular substitute has a non-resorbable porous layer/component, the characteristics of which will provide permanent resistance to the arterial pressure even at extreme levels, and at the same time it does not negatively affect vascular prosthesis healing, or, more specifically, organization, in the body. On its inner and outer sides, this non-resorbable part has a continuous coat of biological resorbable material, which is able to draw closer to blood vessel wall characteristics with its physical characteristics. It is primarily the lower immunogenicity of Czech carp collagen compared with previously used materials - bovine collagen, and salmon and other fish collagen. In addition, collagen obtained from cold-blooded animals eliminates the risk of zoonosis transmission. Collagen obtained from local freshwater fish is cheaper than saltwater fish collagen.

Applying the outer collagen layer or coat onto the non-resorbable layer, knitted fabric, is an indisputable advantage. The wettable or contact surface of knitted fabric fibres takes a large total surface as compared with the compact biological outer collagen layer. The size of the contact prosthesis surface is directly related to the risk of graft infection. The application of this outer collagen layer or coat, which may be enriched with drugs such as antibiotics, significantly decreases the risk of substitute infection. The once infected substitute is always intended for explantation because infection cannot be eliminated from the artificial surface of the substitute (knitted fabric). In addition, the outer layer is a safeguard (barrier) against blood leakage through the prosthesis wall if the inner layer integrity gets damaged. On the other hand, the outer side of the substitute must be resorbed fairly soon in order not to hamper healing the prosthesis in the surrounding tissue. Both biological components have such properties to be controllably resorbable as well as to enable their enriching with pharmacodynamically active substances. All materials used for the composite prosthesis are sterilizable by irradiation with standard expiration time of commonly used vascular prostheses.

We expect this type of vascular substitute to be used especially in the area of low flows, in particular crural arterial vessels with low-diameter target arteries. The indication for distal reconstruction of this type is primarily for patients with critical limb ischaemia (presence of trophic disorder) suffering from the most severe form of peripheral atherosclerosis and diabetes with organ complications.

In general, this vascular substitute is a composite tube that may be used as a substitute for any tubular structures with low inner diameters under 6 mm, and intended for the transport of fluids, i.e. liquids or gases, blood, for example, at low flow rates, in particular under 100 ml/min, and internal pressures inside the tube of 450 mmHg = 60 kPa = 0.6 bar = 8.7 psi.

The process for producing the vascular substitutes is readily reproducible.

### Description of the Drawings

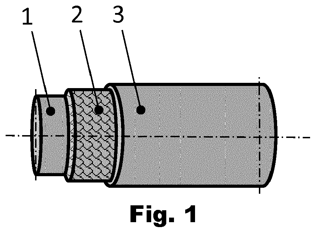

The invention will be described in more detail on an exemplary system using the attached
Fig. 1 showing a schematic broken-out section of the vascular substitute.

### Description of the Embodiments

The exemplary laminated composite vascular substitute, for flow rates under 100 ml/min and inner diameters under 6 mm, contains an inner resorbable collagen compound layer 1 overlaid with a non-resorbable knitted fabric layer 2 overlaid with an outer resorbable collagen compound layer 4. The exemplary monolayer composite vascular substitute, for flow rates under 100 ml/min and inner diameters under 6 mm, contains a non-resorbable layer 2, which is integrated in a single collagen compound layer during the fabrication process. This creates a coat of collagen compound on the inner and outer sides of the non-resorbable layer 2, knitted fabric. The collagen compound is freshwater fish collagen from the skin of Czech carp.

The non-resorbable layer 2 is polyester knitted fabric. One fibre of the non-resorbable layer 2 consists of 2 to 5 monofilaments combined in one bundle before knitting. The knitted fabric is non-folded.

The exemplary method for producing a laminated vascular substitute consists in that the collagen compound with a collagen concentration of 8% by weight is kept at 21°C for 24 hours, and then it is extruded to make the inner resorbable layer 1 in the form of a tube that is dried at the room temperature until pliable. Then the inner resorbable layer 1 is overlaid with the non-resorbable knitted fabric layer 2, to which the outer resorbable layer 3 of collagen compound is applied, with a collagen concentration of 7% by weight.

Another exemplary method for producing a monolayer vascular substitute consists in that the collagen compound with a collagen concentration of 8% by weight is kept at 21 °C for 24 hours, and then the non-resorbable knitted fabric layer 2 is subsequently integrated in one collagen compound layer during the fabrication process such as extrusion. This creates a coat of collagen compound on the inner and outer sides of the non-resorbable layer 2, knitted fabric. The vascular substitutes are dried at 21 °C for 24 hours until pliable. The vascular substitutes are subsequently hardened with 2% resin for 5 minutes and then they are dried and softened up in a 20% glycerine bath for 20 minutes.

The collagen compound is enriched with evenly dispersed pharmacologically effective substances. A collagen compound solution with a collagen concentration of 2% by weight may be applied between the inner resorbable layer 1 and the non-resorbable layer 2.

The vascular substitutes prepared in this way are cut to length and individually wrapped in double sterilization packaging and sterilized by gamma radiation dose of 25 to 30 kGy.

The vascular substitutes are expected to be fabricated in standard sizes for low flow rates, i.e. with diameters of 3 to 6 mm and lengths of 100 to 800 mm. These dimensions correspond to the use for distal anastomosis placement, especially on the arteria poplitea (P3) and crural arteries - arteria fibulars, arteria tibialis posterior and arteria tibialis anterior. The short type of the prosthesis can be used for substitutes in the femoropopliteal region.

### Experimental confirmation:

The patency of this vascular substitute type was demonstrated on an experimental set - ovine model - with excellent patency results in medium-term monitoring, with monitoring low flows through the prosthesis. Subsequently, the prostheses were explanted and subject to histological examination.

**Table 1: Animals used.**

| Animal - sheep # | 1 | 2 | 3 | 4 | 5 | 6 | 7 |
|---|---|---|---|---|---|---|---|
| Weight (kg) | 30 | 30 | 62 | 48 | 53 | 65 | 55 |
| Sex | M | M | F | F | F | F | F |
| Duration of surgery (hrs) | 3.8 | 3.5 | 3.8 | 3 | 3.2 | 3 | 3.4 |
| Monitoring (days) | 379 | 53^{a)} | 308 | 99 | 211 | 160 | 161 |

**Table 2: Characteristics of the set.**

| **No.** | **Side** | **Anastomosis** | **Graft length (mm)** | **Initial flow rate (ml/min)** | **Narrowed flow rate (ml/min)** | **Location of narrowing** | **Final flow rate^{b)} (ml/min)** |
|---|---|---|---|---|---|---|---|
| **1** | dx^{a)} | - | - | - | - | - | - |
| | sin | ETE | 45 | 170 | - | No | 220 |
| **2** | dx | ETE | 45 | 230 | 120 | Inflow ACC | 130 |
| | sin | ETE | 50 | 230 | - | No | 180 |
| **3** | dx | ETE | 40 | 160 | 70 | Prox. and dist. prosthesis end | 160 |
| | sin | ETS | 35 | 160 | - | ACC closed between ligatures | 140 |
| **4** | dx | ETS | 65 | 250 | 100 | ACC closed between ligatures + sling in the outflow ACC | 0 |
| | sin | ETE | 75 | 255 | - | No | ~0 |
| **5** | dx | ETS | 60 | 340 | 120 | ACC closed between ligatures + sling in the outflow ACC | 168 |
| | sin | ETE | 60 | 270 | - | No | 170 |
| **6** | dx | ETE | 60 | 220 | 250 | Outflow ACC narrowing | 160 |
| | sin | ETE | 65 | 480 | - | No | 172 |
| **7** | dx | ETS | 50 | 240 | 145 | ACC closed between ligatures + sling in the outflow ACC | 170 |
| | sin | ETE | 60 | 425 | - | ACC closed between ligatures | 237 |

| | | | | | | | |
|---|---|---|---|---|---|---|---|
| ACC: arteria carotis communis, ETE: end-to-end anastomosis, ETS: end-to-side anastomosis, dx: right-hand, sin: left-hand. | | | | | | | |

### Industrial Applicability

The composite vascular substitute of this invention can be used particularly for distal reconstructions on the lower-limb arteries, i.e. as distal femoropopliteal by-passes, crural by-passes, etc. In general, these are composite tubes that may be used as substitutes for any tubular structures with low inner diameters under 6 mm, and intended for the transport of fluids, i.e. liquids or gases, blood, for example, at low flow rates of under 100 ml/min, and internal pressures inside the tube of 450 mmHg = 60 kPa = 0.6 bar = 8.7 psi.

## Claims

1. A composite vascular substitute, for flow rates under 100 ml/min and inner diameters 3 to 6 mm, ***characterized in that*** it contains a non-folded non-resorbable knitted fabric layer (2) integrated in fish collagen compound from skin of Czech carp, wherein one fibre of the non-resorbable layer (2) consists of 2 to 5 monofilaments combined in one bundle before knitting, and the length of the vascular substitute is 100 to 800 mm.

2. The composite vascular substitute according to Claim 1, ***characterized in that*** the non-resorbable knitted fabric compound (2) is placed on a self-supporting resorbable collagen compound layer (1), wherein the non-resorbable layer (2) is covered with an intermediate collagen compound layer (4) overlaid with an outer, resorbable collagen compound layer (3).

3. The composite vascular substitute according to Claims 1 or 2, ***characterized in that*** the non-resorbable layer (2) is polyester knitted fabric.

4. A method for producing the vascular substitute according to any of the preceding Claims 1 to 3, ***characterized in that*** the collagen compound with a collagen concentration of 4 to 12% by weight is kept at 15 to 30 °C for 18 to 30 hours, and then the non-resorbable knitted fabric layer (2) is integrated in one anisotropic collagen compound layer by extrusion with collagen fibre bundle rotation to create a collagen compound coating on the inner and the outer sides of the non-resorbable knitted fabric layer (2).

5. A method for producing the vascular substitute according to any of the preceding Claims 1 to 3, ***characterized in that*** the collagen compound with a collagen concentration of 4 to 12% by weight is kept at 15 to 30 °C for 18 to 30 hours, and then the collagen compound is extruded to make the inner resorbable layer (1) in the form of a tube that is dried at the room temperature until pliable, and the inner resorbable layer (1) is overlaid with the non-resorbable knitted fabric layer (2), on which the outer resorbable layer (3) of collagen compound with a collagen concentration of 3 to 12% by weight is extruded, and the vascular substitutes are dried at 15 to 30 °C for 15 to 30 hours until pliable, and then the vascular substitutes are hardened with 1.5 to 3.2% resin for 3 to 10 minutes and then they are dried and softened up in a 15 to 30% glycerine bath for 15 to 25 minutes.

6. The method for producing the vascular substitute according to Claims 4 or 5, ***characterized in that*** the collagen compound is enriched with evenly dispersed pharmacologically effective substances.

7. The method for producing the vascular substitute according to Claims 4, 5 or 6, ***characterized in that*** a collagen compound solution with a collagen concentration of 1 to 3% by weight may be applied between the inner resorbable layer (1) and the non-resorbable layer (2).

8. The method for producing the vascular substitute according to any of the preceding claims 4-7, ***characterized in that*** the vascular substitute specimens are individually wrapped in double sterilization packaging and sterilized by gamma radiation dose of 25 to 30 kGy.

## Patentansprüche

1. Ein Komposit-Blutgefäßersatz für Durchflussraten unter 100 ml/min und mit einem Innendurchmesser im Bereich von 3 bis 6 mm, ***dadurch gekennzeichnet, dass*** er eine nicht gewrappte, unabsorbierbare Gestrickschicht (2) enthält, die in eine aus einer Haut des Tschechischen Karpfens erhaltene Fischkollagenmasse integriert ist, wobei eine Faser der unabsorbierbaren Schicht (2) aus 2 bis 5, vor dem Stricken zu einem Bündel zusammengefassten Monofilamenten besteht, und wobei die Länge des Blutgefäßersatzes 100 bis 800 mm beträgt.

2. Der Komposit-Blutgefäßersatz nach Anspruch 1, ***dadurch gekennzeichnet, dass*** die unabsorbierbare Gestrickschicht (2) auf einer selbsttragenden inneren absorbierbaren Kollagenmasse-Schicht (1) aufgebracht ist, wobei die unabsorbierbare Schicht (2) von einer Zwischenschicht aus einer Kollagenmasse bedeckt ist, auf der eine äussere absorbierbare Kollagenmasse-Schicht (3) aufgebracht ist.

3. Der Komposit-Blutgefäßersatz nach Anspruch 1 oder 2, ***dadurch gekennzeichnet, dass*** die unabsorbierbare Schicht (2) ein Polyestergestrick ist.

4. Ein Verfahren zur Herstellung des Blutgefäßersatzes nach einem der Ansprüche 1 bis 3, ***dadurch gekennzeichnet, dass*** eine Kollagenmasse mit einer im Bereich von 4 bis 12 Gew. % liegenden Konzentration an Kollagen 18 bis 30 Stunden lang bei einer Temperatur von 15 bis 30 °C temperiert wird, worauf die unabsorbierbare Gestrickschicht (2) durch das unter Verdrehung der Bündel von Kollagenfasern stattfindende Extrudieren in eine einzelne Schicht einer anisotropen Kollagenmasse, unter Bildung eines aus der Kollagenmasse bestehenden Belags innerseits sowie ausserseits der unabsorbierbare Gestrickschicht (2), integriert wird.

5. Ein Verfahren zur Herstellung des Blutgefäßersatzes nach einem der Ansprüche 1 bis 3, ***dadurch gekennzeichnet, dass*** eine Kollagenmasse mit einer im Bereich von 4 bis 12 Gew. % liegenden Konzentration an Kollagen 18 bis 30 Stunden lang bei einer Temperatur von 15 bis 30 °C temperiert wird, worauf aus der Kollagenmasse die innere absorbierbare Schicht (1) in der Form einer Röhre extrudiert wird, die bei einer Raumtemperatur bis zum Erreichen eines schmiegsamen Zustands getrocknet wird, worauf die innere absorbierbare Schicht (1) mit der unabsorbierbaren Gestrickschicht (2) überzogen wird, auf die anschliessend äussere absorbierbare Schicht (3) aus einer Kollagenmasse mit einer im Bereich von 3 bis 12 Gew. % liegenden Konzentration an Kollagen extrudiert wird, worauf der Blutgefäßersatz 15 bis 30 Stunden lang bei einer Temperatur von 15 bis 30 °C bis zum Erreichen eines schmiegsamen Zustands getrocknet wird und nachfolgend der Blutgefäßersatz 3 bis 10 Minuten lang mittels eines Harzes in einer Menge von 1,5 bis 3,2 % ausgehärtet wird und nach weiterer Trocknung 15 bis 25 Minuten lang in einem 15 bis 30 % an Glyzerin enthaltenden Bad aufgeweicht wird.

6. Das Verfahren zur Herstellung des Blutgefäßersatzes nach einem der Ansprüche 4 oder 5, ***dadurch gekennzeichnet, dass*** die Kollagenmasse gleichmäßig mit dispergierten pharmakologisch wirksamen Stoffen angereichert ist.

7. Das Verfahren zur Herstellung des Blutgefäßersatzes nach einem der Ansprüche 4, 5 oder 6, ***dadurch gekennzeichnet, dass*** eine Lösung einer Kollagenmasse mit einer im Bereich von 1 bis 3 Gew. % liegenden Konzentration an Kollagen zwischen die innere absorbierbare Schicht (1) und die unabsorbierbare Schicht (2) aufgetragen werden kann.

8. Das Verfahren zur Herstellung des Blutgefäßersatzes nach einem der Ansprüche 4 bis 7, ***dadurch gekennzeichnet, dass*** die Proben des Blutgefäßersatzes einzeln in eine doppelte Sterilisationshülle verpackt und anschliessend mit Gammastrahlung in einer im Bereich von 25 bis 30 kGy liegenden Dosis sterilisiert werden.

## Revendications

1. Remplacement vasculaire composite pour l'écoulement sous 100 ml/min et pour un diamètre intérieur de 3 à 6 mm, **caractérisé en ce qu'**il comprend une couche (2) tricotée non pliée et non résorbable, intégrée dans du composé de collagène de poisson issu de la peau de la carpe tchèque, où une fibre de la couche (2) non résorbable consiste en 2 à 5 monofilaments combinées dans un faisceau avant du tricotage, et la longueur du remplacement vasculaire et de 100 à 800 mm.

2. Remplacement vasculaire composite selon la revendication 1, **caractérisé en ce que** la couche (2) tricotée non résorbable est placée sur une couche (1) autoportante résorbable de composé de collagène, où la couche (2) non résorbable est recouverte d'une couche (4) intermédiaire de composé de collagène, recouverte d'une couche (3) extérieure résorbable de composé de collagène.

3. Remplacement vasculaire composite selon la revendication 1 ou 2, **caractérisé en ce que** la couche (2) non résorbable est un tissu tricoté de polyester.

4. Procédé pour la production du remplacement vasculaire selon l'une quelconque des revendications précédentes 1 à 3, **caractérisé en ce que** le composé de collagène ayant une concentration de collagène comprise entre 4 à 12% en poids est maintenu à une température de 15 à 30 °C pendant 18 à 30 heures, puis la couche (2) du tissu tricoté non résorbable est intégrée dans une couche anisotrope de composé de collagène par extrusion avec une rotation d'un faisceau de fibres de collagène afin de créer un revêtement de composé de collagène sur le coté intérieur et le coté extérieur de la couche (2) de tissu tricoté non résorbable.

5. Procédé pour la production du remplacement vasculaire selon l'une quelconque des revendications précédentes 1 à 3, **caractérisé en ce que** le composé de collagène ayant une concentration de collagène comprise entre 4 à 12% en poids est maintenu à une température de 15 à 30 °C pendant 18 à 30 heures, et puis le composé de collagène est extrudé pour former la couche (1) résorbable intérieure dans une forme d'une tube qui est séchée à température ambiante jusqu'à ce qu'elle est souple, et la couche (1) résorbable intérieure est recouverte de la couche (2) de tissu tricoté non résorbable, sur laquelle la couche (3) résorbable extérieure de composé de collagène ayant une concentration de collagène comprise entre 3 à 12 % en poids est extrudée, et les remplacements vasculaires sont séchés à température de 15 à 30 °C pendant 15 à 30 heures jusqu'à ce qu'ils sont souples, et puis les remplacements vasculaires sont durcis par de la résine de 1.5 à 3.2% pendant 3 à 10 minutes, et puis ils sont séchés et ramollis dans un bain de glycérine de 15 à 30 % pendant 15 à 25 minutes.

6. Procédé pour la production du remplacement vasculaire selon la revendication 4 ou 5, **caractérisé en ce que** le composé de collagène est enrichi de substances pharmacologiquement effectives et uniformément dispersées.

7. Procédé pour la production du remplacement vasculaire selon la revendication 4, 5 ou 6, **caractérisé en ce qu'**une solution de composé de collagène ayant une concentration de collagène comprise entre 1 à 3% en poids peut être appliquée entre la couche (1) résorbable intérieure et la couche (2) non résorbable.

8. Procédé pour la production du remplacement vasculaire selon l'une quelconque des revendications précédentes 4 à 7, **caractérisé en ce que** les spécimens du remplacement vasculaire sont individuellement emballés dans un emballage de stérilisation double et stérilisés par une dose de rayonnement gamma de 25 à 30 kGv.
